# Europäisches Patentamt
## European Patent Office
## Office européen des brevets

(19)

(11) Veröffentlichungsnummer: **0 133 614**
**B1**

(12) # EUROPÄISCHE PATENTSCHRIFT

(45) Veröffentlichungstag der Patentschrift:
15.02.89

(51) Int.'Cl.⁴: **C 02 F 3/34, C 02 F 1/48**

(21) Anmeldenummer: 84890114.6

(22) Anmeldetag: 19.06.84

(54) **Verfahren zur biologischen Umsetzung von Substraten.**

(30) Priorität: 05.07.83 AT 2471/83

(43) Veröffentlichungstag der Anmeldung:
27.02.85 Patentblatt 85/9

(45) Bekanntmachung des Hinweises auf die Patenterteilung:
15.02.89 Patentblatt 89/7

(84) Benannte Vertragsstaaten:
AT BE CH DE FR GB IT LI LU NL SE

(56) Entgegenhaltungen:
EP-A-0 082 809
FR-A-2 259 904
FR-A-2 336 480
GB-A-1 038 405

(73) Patentinhaber: VOEST- ALPINE Aktiengesellschaft,
Friedrichstrasse 4, A-1011 Wien (AT)

(72) Erfinder: Treso, Bertalan, Kunigundenweg 11a,
A-8707 Leoben (AT)
Erfinder: Puschenjak, Erwin, Bahnhofstrasse 1,
A-8714 Kraubath (AT)
Erfinder: Hanke, Reinhart, Dipl.- Ing.,
Annaberggasse 2, A-8700 Leoben (AT)

(74) Vertreter: Haffner, Thomas M., Dr.,
Patentanwaltskanzlei Dipl.- Ing. Adolf
Kretschmer Dr. Thomas M. Haffner
Schottengasse 3a, A-1014 Wien (AT)

EP 0 133 614 B1

## Beschreibung

Die Erfindung bezieht sich auf ein Verfahren zur biologischen Umsetzung von Substraten, bei welchem Substrat und mikrobielles Material gegebenenfalls nach einer Vorreinigung des Substrates miteinander vermischt und das auf diese Weise erhaltene mikrobielle System unter Reaktionsbedingungen gehalten wird.

Unter mikrobiellen Systemen werden im folgenden Bakterien, Hefen u.dgl., vermengt mit einem entsprechenden Substrat verstanden. Derartige mikrobielle Systeme werden häufig auch als Biomasse bezeichnet, welche als in sich geschlossenes System eine biologische Umsetzung vornimmt. Mikrobielle Systeme sind insbesondere Belebt- oder Faulschlamm. Ebenso sollen die mikrobiellen Biozönosen, wie sie beispielsweise bei der Essigsäure- oder Alkoholgärung vorliegen unter dem Begriff der mikrobiellen Systeme subsumiert werden. Derartige mikrobielle Systeme nehmen das Substrat üblicherweise durch Absorption an der Zellmembran auf. Im Bereich dieser Zellmembran finden auch die ersten Abbauvorgänge statt. Die Geschwindigkeit der Aufnahme des Substrates wird unter anderem von den Konzentrationen der primären Metaboliten im Zellplasma bestimmt. Je nach Art der eingesetzten Mikroorganismen ist ein derartiges mikrobielles System in der Lage, eine bestimmte biologische Umsetzung durchzuführen bzw. bestimmte im Substrat enthaltene Substanzen zu verarbeiten.

Zur Verbesserung der Beladung der Mikroorganismen mit dem aufzuarbeitenden Substrat werden Polyelektrolyte zugesetzt, welche offensichtlich einen Einfluß auf die Transportvorgänge durch die Zellmembran ausüben. Der Zusatz größerer Mengen von Polyelektrolyten zeigt aber häufig auch negative Einflüsse auf die Tätigkeit der Mikroben, welche damit erklärt werden können, daß die Transportvorgänge durch die Zellmembran blockiert werden. Eine Reihe von mikrobiellen Systemen ist überaus schwer flotierbar und es gelingt daher die Abtrennung des aufzuarbeitenden Substrates nur in unbefriedigendem Ausmaß. Der Zusatz größerer Mengen an Polyelektrolyten hat nachteilige Auswirkungen auf die Abwasserbelastung. Darüberhinaus verläuft eine Reihe an biologischen Umsetzungen in derartigen mikrobiologischen Systemen überaus langsam, weil es nicht gelingt, eine rasche Aufnahme des abzubauenden Substrates durch die Mikroben zu erreichen.

Die Erfindung zielt nun darauf ab, die Geschwindigkeit der Beladung von mikrobiellen Material durch das Substrat zu erhöhen, die Menge an zuzusetzendem Polyelektrolyten wesentlich zu verringern und die Flotierbarkeit der beladenen Biomasse zu verbessern. Zur Lösung dieser Aufgabe besteht die Erfindung im wesentlichen darin, daß nach dem Vermischen des Substrates mit dem mikrobiellen Material ein elektrisches Feld unter Anwendung von pulsierendem Gleichstrom im Mischraum und/oder in einer Flotationseinrichtung angelegt wird, und daß nachfolgend eine biologische Umsetzung ohne Anlegen eines elektrischen Feldes vorgenommen wird. Es hat sich hierbei als wesentlich herausgestellt, daß ein pulsierender Gleichstrom zum Aufbau des elektrischen Feldes verwendet wird. Ein derartiger pulsierender Gleichstrom kann auch ein Schwellstrom sein, welcher ausgehend von einem minimalen Wert Halbwellen nur einer Polarität aufweist. Durch die Anwendung eines derartigen elektrischen Feldes nach dem Vermischen des Substrates mit dem mikrobiellen Material wurde überraschenderweise gefunden, daß das Substrat von den Mikroben verstärkt aufgenommen wird, wobei die zu erwartenden Konzentrationen der primären Metaboliten offenbar keinen nennenswerten Einfluß auf die Geschwindigkeit der Substratresorption zeigten. Es ist zwar bereits bekannt, Abwässer vor einer biologischen Reinigung einer Elektrolyse zu unterwerfen. Bisher bestand jedoch offensichtlich ein Vorurteil der Fachwelt, die Biomasse selbst unter die Einwirkung eines elektrischen Feldes zu setzen. Die überraschenden Effekte der Einwirkung des elektrischen Feldes mit pulsierendem Gleichstrom auf die Biomasse können möglicherweise dadurch erklärt werden, daß die Ladungsverteilung an der Zellmembran unter dem Einfluß des elektrischen Feldes verändert wird, wodurch die Transportvorgänge durch die Zellmembran wesentlich beschleunigt werden. Die Überlegenheit der Verwendung eines pulsierenden Gleichstromes gegenüber glattem Gleichstrom konnte experimentell belegt werden, wobei eine exakte Erklärung hierfür nicht gefunden werden konnte. Für die Überlegenheit gegenüber der Verwendung von Wechselstrom mit positiven und negativen Halbwellen dürfte die Tatsache sprechen, daß die Polaritätsumkehrung beim Nulldurchgang derartiger Halbwellen die Aktivierung der Zellmembrane wiederum stört. Durch das Anlegen eines derartigen elektrischen Feldes wurde eine Mehraufnahme an Substrat durch die Mikroben beobachtet und es wurde vereinzelt sogar beobachtet, daß Mikroben nunmehr auch Substanzen aufnehmen, welche sie üblicherweise nicht verarbeiten. So wurde beispielweise bei der Spezies Sacharomycetes cerevisiae (Bierbrauhefe) beobachtet, daß sie neben den üblicherweise von dieser Spezies verwerteten Hexosen im elektrischen Feld auch Pentosen unter anaeroben Bedingungen aufnimmt. In der Folge wurde beobachtet, daß von dieser Spezies unter der Einwirkung des elektrischen Feldes Xylose zu Alkohol vergoren wird. Bei vielen anderen Mikroben konnte beobachtet werden, daß nicht nur größere Mengen an Substrat in kürzerer Zeit von den Mikroben aufgenommen werden konnten, sondern daß auch die Abtrennbarkeit der Biomasse wesentlich verbessert wurde. Schwer flotierbare mikrobielle Systeme und nicht flotierbare Substanzen konnten in Anwesenheit eines elektrischen Feldes leicht abgetrennt werden und die verbleibende Konzentration im Restwasser nach dem Abtrennen der Biomasse im Anschluß an die Substrataufnahme ergab wesentlich geringere Abwasserwerte als bei den bekannten Verfahren. Das relativ höhere Angebot an Substrat in der Zelle zwingt offensichtlich die Zelle zu beschleunigter Umsetzung und es wurde in der Folge eine wesentlich raschere Umsetzung zu den gewünschten Endprodukten beobachtet. Vor allen Dingen konnte der Bedarf an Polyelektrolyten bei der Flotation wesentlich herabgesetzt werden und die Dauer der Flotation wesentlich verkürzt werden. Beispielsweise wurde beobachtet, daß eine Flotation, welche bei konventionellen Verfahren ohne Anwendung eines elektrischen Feldes eine dreiviertel Stunde bis eine Stunde benötigte, nunmehr unter Anwendung eines elektrischen Feldes in 4 bis 5 Minuten zu Ende geführt werden konnte, wobei eine geringere Konzentration an Substrat im Abwasser verblieb.

Eine Flotation ist im Rahmen von Abwasserreinigungsanlagen besonders vorteilhaft, da die Reinigungsanlage entsprechend dem durchschnittlichen Bedarf ausgelegt wird. Durch die Flotation können Spitzenwerte im Anfall von zu klärendem Abwasser in einfacher Weise dadurch bewältigt werden, daß der in der normal dimensionierten biologischen Reinigungsstufe nicht mehr durchsetzbare flotierte Schlamm als Überschußschlamm ausgetragen und verbrannt wird, oder aber in konzentrierter Form zwischengespeichert wird, um in der Folge in entsprechender Verdünnung der konventionellen biologischen Reinigung unterworfen zu werden. Die Flotationsstufe ermöglicht hierbei vor allen Dingen aufgrund des geringen Zeitbedarfes kurzfristige Regeleingriffe, wie beispielsweise die Abtrennung von Schlamm, welcher nach neuerlicher Belüftung dem Mischbecken rückgeführt werden kann. Auch auf diese Weise gelingt es, dem unterschiedlichen Anfall von zu klärendem Wasser Rechnung zu tragen und einen gleichmäßigen Durchsatz durch die biologische Reinigungsstufe sicherzustellen. Die teilweise Rückführung von flotiertem Schlamm zum neuerlichen Mischen erlaubt es, geringere Mengen an Nährstoff zuzusetzen, da dieser rückgeführte Schlamm speziell bei kommunalen Abwassern einen hohen Nährstoffanteil enthält.

Im Rahmen des erfindungsgemäßen Verfahrens hat es sich als vorteilhaft herausgestellt, für den Aufbau des elektrischen Feldes Klemmenspannungen von 2 - 30 V, vorzugsweise 4 - 12 V, anzuwenden.

Auch wenn der Zusatz von Polyelektrolyten wesentlich verringert werden kann, ist es doch vorteilhaft, insbesondere zur Erzielung einer verbesserten Leitfähigkeit der Lösung der Mischung aus Substrat und mikrobiellen Material vor dem Anlegen des elektrischen Feldes Polyelektrolyte zuzusetzen. Überraschenderweise hat auch der Zusatz von Ionenaustauschern eine Verbesserung der Substrataufnahme und Flotation im elektrischen Feld mit sich gebracht.

Vorzugsweise wird das elektrische Feld intermittierend angelegt, wobei das elektrische Feld zumindest in einem ersten zeitlichen Teilbereich der Umsetzung, in welchem das Substrat durch das mikrobielle Material aufgenommen wird, angelegt wird. Innerhalb dieses ersten zeitlichen Teilbereiches hat sich eine signifikante Verbesserung der Beladung der Mikroben bei gleichzeitiger Verringerung des erforderlichen Zeitbedarfes und bei gleichzeitiger Verbesserung der Flotierbarkeit gezeigt.

Bei Biozönosen, welche nicht unmittelbar nach ihrer Beladung zu starker Gasbildung neigen, ist es vorteilhaft, die Flotation durch elektrolytische Zersetzung zu unterstützen. In diesem Fall wird mit Vorteil so vorgegangen, daß in dem ersten zeitlichen Teilbereich eine Klemmenspannung oberhalb der Zersetzungsspannung der Substrat-Biomasselösung zur Begünstigung der Flotation der mit Substrat beladenen Biomasse angelegt wird. Bei Biozönosen, welche größere Mengen an Gärgasen freisetzen, kann die Klemmenspannung geringer gewählt werden.

In jedem Falle ist es günstig, der Mischung bereits vor dem Anlegen des elektrischen Feldes Nährstoffe zuzusetzen, wobei gegebenenfalls der pH auf den für die biologische Umsetzung günstigsten Wert gebracht werden kann. Die Einstellung des pH-Wertes auf den für die nachfolgende biologische Umsetzung günstigen Wert erlaubt in der Regel eine weitere Herabsetzung der zuzusetzenden Menge an Polyelektrolyt.

In vorteilhafter Weise wird ein elektrisches Feld mit im wesentlichen horizontalen Feldlinien angewendet. Bei einem derartigen elektrischen Feld werden die Elektroden üblicherweise vertikal angeordnet und die an der Oberfläche der Elektroden einsetzende Gasbildung unterstützt die Flotation der Biomasse.

Da die Biomasse während der eigentlichen biologischen Umsetzung, welche als anaerobe oder aerobe Umsetzung geführt werden kann, nicht mehr einem elektrischen Feld unterworfen werden soll und vor allen Dingen um für die nachgeschaltete biologische Umsetzung beliebige Umsetzungsverfahren, wie Gärung, Fäulung, Atmung oder ein übliches Schlammbecken zu verwenden, wird das erfindungsgemäße Verfahren mit Vorteil so ausgeführt, daß die Beladung der Biomasse mit Substrat und die biologische Umsetzung der mit Substrat beladenen Biomasse in gesonderten Behältern vorgenommen wird, wobei das mit Substrat beladene Material abgetrennt und in den Reaktionsraum übergeführt wird und die Biomasse nach der Umsetzung im Reaktionsraum dem Mischraum zurückgeführt wird. Durch diese Verfahrensweise kann den unterschiedlichsten Biomassen Rechnung getragen werden. Während beispielsweise Hefe sehr rasch Kohlendioxid entwickelt und daher in der ersten Phase der Beladung des mikrobiellen Materials mit dem Substrat keine besondere Unterstützung durch elektrolytische Zersetzung für eine rasche und vollständige Flotation erfordert, ist es bei Belebtschlamm, welcher nur schwer flotiert überaus vorteilhaft im ersten zeitlichen Teilbereich der Umsetzung, in welchem die Beladung der Biomasse erfolgt, höhere Spannungen anzulegen um die Flotation zu begünstigen.

Das elektrische Feld kann im Rahmen des erfindungsgemäßen Verfahrens in besonders bevorzugter Weise dadurch angelegt werden, daß im Mischraum und/oder dem Flotationsraum Elektroden in einem Abstand voneinander angeordnet werden, welcher in Abhängigkeit von der gewählten Spannung im Bereich von 2 - 30 V, 0,5 bis 2 mm/V, vorzugsweise etwa 1 mm/V beträgt. Unter Einhaltung dieser Bedingungen konnte ein Optimum in Bezug auf die Beladung der Mikroben mit Substrat und im Bezug auf die Flotation der beladenen Biomasse beobachtet werden. Vorzugsweise wird das Verfahren etwa so durchgeführt, daß der Elektrodenabstand im Flotationsraum gegenüber dem Elektrodenabstand im Mischraum und/oder die Klemmenspannung im Mischraum gegenüber der Klemmenspannung im Flotationsraum größer gewählt wird.

Im Rahmen des Verfahrens können abbaubare Anoden, z. B. aus Aluminium oder Eisen, ebenso wie gegen elektrolytische Zersetzung resistente Anoden eingesetzt werden. Die Abtrennung der beladenen Biomasse kann durch die Verwendung von Flockungshilfsmitteln unterstützt werden. Das stimulierende Effekt des elektrischen Feldes für die Aufnahme von Substrat konnte sowohl für anaerobe als auch für aerobe Umsetzungen gefunden werden.

Mit Vorteil kann das erfindungsgemäße Verfahren kontinuierlich durchgeführt werden, wobei nach einer Vermischung des Substrates mit dem mikrobiellen Material diese Mischung kontinuierlich einem ersten Raum zugeführt wird, in welchem die Beladung des mikrobiellen Materials mit dem Substrat unter Einfluß des elektrischen Feldes erfolgt. In diesem ersten Raum wird auch die Flotation vorgenommen und die Biomasse abgetrennt. Die auf diese Weise abgetrennte und beladene Biomasse kann in einem nachfolgenden Reaktionsraum biologisch umgesetzt werden.

Nach der biologischen Umsetzung kann die Biomasse nach dem Substratabbau im Rahmen des erfindungsgemäßen Verfahrens rückgeführt werden, wobei sich eine besonders ökonomische Verfahrensführung ergibt.

Die Erfindung wird nachfolgend an Hand von Ausführungsbeispielen näher erläutert.

**Beispiel 1:**

Kommunales Abwasser mit einem biologischen Sauerstoffbedarf $BSB_5$ von 550 mg/l wurde mit Belebtschlamm im Verhältnis 1 : 1 gemischt. Der Trockensubstanzgehalt in Belebtschlamm (Rücklaufschlamm) betrug 5,0 g/l. Nach einer Rührzeit bzw. Mischdauer von etwa 2,5 min wurde die Biomasse einem elektrischen Feld unterworfen. Der Elektrodenabstand betrug hierbei 15 mm und es wurde mit einer sekundären Klemmenspannung von 16 V und einer Stromdichte von 0,5 A/dm² gearbeitet. Die Aufenthaltszeit im elektrischen Feld betrug 6 min. Anschließend wurde eine Konzentration des flotierten Schlammes von 25 g Trockensubstanz/kg sowie eine Belastung des verbleibenden Restwassers $BSB_5$ von 80 mg/l festgestellt.

Zu Vergleichszwecken wurde der gleiche Versuch ohne Anwendung eines elektrischen Feldes wiederholt. Ausgehend von einem wie zuvor hergestellten Gemisch von Abwasser und Belebtschlamm im Verhältnis 1 : 1 wurde bei einem Ausgangsvolumen von 1 l eine Sedimentationsgeschwindigkeit von 0,24 m/h entsprechend einer Aufenthaltszeit von 2 h festgestellt. Das Schlammvolumen betrug 330 ml, wobei die Restbelastung im Dekantat ausgedrückt in $BSB_5$ 210 mg/l betrug. Aus diesem Vergleich ergibt sich klar die wesentlich geringere Belastung des Restwassers und die wesentlich raschere Beladung der Biomasse unter der Einwirkung des elektrischen Feldes.

**Beispiel 2:**

Es wurde eine Zuckerlösung einer Konzentration von 200 g/l mit Hefeschlamm vermischt. Die Konzentration betrug 100 g Trockensubstanz/l. Nach einer Rührzeit bzw. Mischdauer von 10 min wurde ein elektrisches Feld angelegt, wobei der Elektrodenabstand 5 mm betrug und die Klemmenspannung zwischen 4 und 5 V gewählt wurde. Die Stromdichte wurde zwischen 0,5 und 2 A/dm² gewählt, wobei die Aufenthaltszeit im elektrischen Feld mit 10 min gewählt wurde. Bei dieser Verfahrensweise konnte eine Zuckerkonzentration im flotierten Schlamm von 163 g/l und eine Zuckerkonzentration im Flotationswasser von 37 g/l gefunden werden.

Zu Vergleichszwecken wurde die gleiche Verfahrensweise ohne Anwendung eines elektrischen Feldes, jedoch mit Zusatz von erheblichen Mengen eines Polyelektrolyten wiederholt. Ausgehend von der oben beschriebenen Mischung der Zuckerlösung mit dem Hefeschlamm wurde nach Zusatz von 150 g/l Polyelektrolyt und einer Rührzeit bzw. Mischdauer von 10 min eine Flotation mit dem durch die Gärung gebildeten $CO_2$ über 15 min durchgeführt. Trotz der gegenüber der Flotation im elektrischen Feld längeren Flotationsdauer wurde eine Zuckerkonzentration im flotierten Schlamm von lediglich 138 g/l und eine Zuckerkonzentration im Flotationswasser von 62 g/l gefunden.

Als besonders vorteilhaft hat sich ein pulsierender Gleichstrom mit einer Pulsfrequenz von etwa 100 Herz erwiesen.

Während der Flotation werden hierbei innerhalb kürzester Zeit gut abtrennbare Flocken gebildet. Diese Flocken zeigen eine hervorragende Beladung. Die Flockenstruktur ist allerdings in einem Belebtschlammbecken für die weitere biologische Umsetzung nicht unbedingt von Vorteil und es kann vorteilhaft sein, die Flockenstruktur vor der biologischen Umsetzung durch Belüftung zu zerstören. Eine derartige Schlammbelüftung kann auch dazu herangezogen werden, einen Teil des Schlammes dem Mischbecken wiederum zurückzuführen und auf diese Weise einen in Bezug auf biologische Trockensubstanz konstanten Schlamm der biologischen Aufbereitung zuzuführen. Das bei der Flotation verbleibende Abwasser kann in der Regel unmittelbar in den Vorfluter abgeleitet werden. Die Trennung von Mischung, Flotation und biologischer Umsetzung erlaubt eine Reihe von Kreislaufführungen, wobei insbesondere die zwischengeschaltete Schlammbelüftung für die Rückführung eines Teiles des Schlammes zum Mischbecken oder für die Zerstörung der Flockenstruktur zur Verbesserung der nachfolgenden Umsetzung besonders vorteilhaft ist. Teile des belüfteten Schlammes können auch gemeinsam mit dem dem Vorklärbecken oder einem Nachklärbecken entnommenen Schlamm einer weiteren Schlammbehandlung unterworfen werden, wodurch eine einfache Anpassung an die bestehende Anlagenkapazität ermöglicht wird.

**Patentansprüche**

1. Verfahren zur biologischen Umsetzung von Substraten, bei welchem Substrat und mikrobiellen Material gegebenenfalls nach einer Vorreinigung des Substrates miteinander vermischt und das auf diese Weise erhaltene mikrobielle System unter Reaktionsbedingungen gehalten wird, dadurch gekennzeichnet, daß nach dem Vermischen des Substrates mit dem mikrobiellen Material ein elektrisches Feld unter Anwendung von pulsierendem Gleichstrom im Mischraum und/oder in einer Flotationseinrichtung angelegt wird, und daß nachfolgend eine biologische Umsetzung ohne Anlegen eines elektrischen Feldes vorgenommen wird.

2. Verfahren nach Anspruch 1, dadurch gekennzeichnet, daß für den Aufbau des elektrischen Feldes Klemmenspannungen von 2 - 30 V, vorzugsweise 4 - 12 V, angewendet werden.

3. Verfahren nach Anspruch 1 oder 2, dadurch gekennzeichnet, daß der Mischung aus Substrat und mikrobiellen Material vor dem Anlegen des elektrischem Feldes Polyelektrolyte und/oder Ionenaustauscher zugesetzt werden.

4. Verfahren nach einem der Ansprüche 1, 2 oder 3, dadurch gekennzeichnet, daß das elektrische Feld intermittierend angelegt wird.

5. Verfahren nach einem der Ansprüche 1 bis 4, dadurch gekennzeichnet, daß das elektrische Feld zumindest in einem ersten zeitlichen Teilbereich der Umsetzung, in welchem das Substrat durch das mikrobielle Material aufgenommen wird, angelegt wird.

6. Verfahren nach Anspruch 5, dadurch gekennzeichnet, daß in dem ersten zeitlichen Teilbereich eine Klemmenspannung oberhalb der Zersetzungsspannung der Substrat-Biomasselösung zur Begünstigung der Flotation der mit Substrat beladenen Biomasse angelegt wird.

7. Verfahren nach einem der Ansprüche 1 bis 6, dadurch gekennzeichnet, daß der Mischung vor Anlegen des elektrischen Feldes Nährstoffe zugesetzt werden, wobei gegebenenfalls der pH auf einem gewünschten Wert gebracht wird.

8. Verfahren nach einem der Ansprüche 1 bis 7, dadurch gekennzeichnet, daß ein elektrisches Feld mit im wesentlichen horizontalen Feldlinien angewendet wird.

9. Verfahren nach einem der Ansprüche 1 bis 8, dadurch gekennzeichnet, daß die Beladung der Biomasse mit Substrat und die biologische Umsetzung der mit Substrat beladenen Biomasse in gesonderten Behältern vorgenommen wird, wobei das mit Substrat beladene Material abgetrennt und in einen Reaktionsraum, insbesondere ein Belebtschlammbecken, übergeführt wird und die Biomasse nach der Umsetzung im Reaktionsraum, gegebenenfalls nach einer Belüftung, dem Mischraum zurückgeführt wird.

10. Verfahren nach einem der Ansprüche 1 bis 9, dadurch gekennzeichnet, daß im Mischraum und/oder der Flotationseinrichtung die Elektroden in einem Abstand voneinander angeordnet werden, welcher in Abhängigkeit von der gewählten Spannung im Bereich von 2 - 30 V 0,5 - 2 mm/V, vorzugsweise etwa 1 mm/V beträgt.

11. Verfahren nach einem der Ansprüche 1 bis 10, dadurch gekennzeichnet, daß der Elektrodenabstand in der Flotationseinrichtung gegenüber dem Elektrodenabstand im Mischraum und/oder die Klemmenspannung im Mischraum gegenüber der Klemmenspannung im Reaktionsraum größer gewählt wird.

12. Verfahren nach einem der Ansprüche 1 bis 11, dadurch gekennzeichnet, daß das elektrische Feld in dem vom Reaktionsraum, insbesondere dem Belebtschlamm- bzw. Belüftungsbecken gesonderten Mischbecken bzw. Flotationsbecken, über einen Zeitraum von 2 - 10 min, vorzugsweise etwa 5 min, aufrechterhalten wird.

## Claims

1. A method for the biological conversion of substrates, wherein the substrate and microbial material are mixed together optionally after preliminary purification of the substrate, and the microbial system obtained in this way is maintained under reaction conditions, characterised in that after the mixing of the substrate with the microbial material an electric field is applied, using pulsating direct current, in the mixing chamber and/or in a flotation apparatus, and in that subsequently a biological conversion is carried out without application of an electric field.

2. A method according to Claim 1, characterised in that terminal voltages of 2 - 30 V, preferably 4 - 12 V, are used to provide the electric field.

3. A method according to Claim 1 or 2, characterized in that polyelectrolytes and/or ion exchangers are added to the mixture of substrate and microbial material before the application of the electric field.

4. A method according to any one of Claims 1, 2 or 3, characterized in that the electric field is applied intermittently.

5. A method according to any one of Claims 1 to 4, characterized in that the electric field is applied at least in an initial period of the conversion, in which the substrate is absorbed by the microbial material.

6. A method according to Claim 5, characterized in that in the initial period a terminal voltage is applied above the decomposition voltage of the substrate biomass solution so as to assist the flotation of the biomass laden with substrate.

7. A method according to any one of Claims 1 to 6, characterised in that nutrients are added to the mixture before the application of the electric field, the pH value optionally being brought to a desired value.

8. A method according to any one of Claims 1 to 7, characterised in that an electric field is used having substantially horizontal field lines.

9. A method according to any one of Claims 1 to 8, characterised in that the loading of the biomass with substrate and the biological conversion of the biomass laden with substrate is carried out in separate vessels, the material laden with substrate being separated and transferred to a reaction chamber, in particular an activated sludge tank, and after the conversion in the reaction chamber the biomass is fed back to the mixing chamber, optionally after aeration.

10. A method according to any one of Claims 1 to 9, characterized in that the electrodes are arranged spaced apart in the mixing chamber and/or flotation apparatus and the spacing, which depends on the chosen voltage within the range of from 2 - 30 V, is 0.5 - 2 mm/V, preferably approximately 1 mm/V.

11. A method according to any one of Claims 1 to 10, characterized in that the electrode spacing in the flotation apparatus is chosen to be greater than the electrode spacing in the mixing chamber and/or the terminal voltage in the mixing chamber is chosen to be greater than the terminal voltage in the reaction chamber.

12. A method according to any one of Claims 1 to 11, characterized in that the electric field is maintained for a period of 2 - 10 min., preferably approximately 5 min., in the mixing chamber and/or flotation chamber which is separated from the reaction chamber, in particular the activated sludge tank or aeration tank.

## Revendications

1. Procédé pour la transformation biologique de substrats dans lequel le substrat et le matériel microbien sont mélangés, le cas échéant après épuration préalable du substrat, et le système microbien ainsi obtenu est maitenu dans des conditions de réaction, caractérisé en ce qu'un courant continu pulsé est appliqué dans l'enceinte de mélange et/ou dans un dispositif de flottation, après mélange du substrat et du matériel microbien, et en ce qu'il est procédé ensuite à une transformation biologique, sans application d'un champ électrique.

2. Procédé selon la revendication 1, caractérié en ce que l'on applique des tensions aux bornes comprises entre 2 et 30 V, de préférence entre 4 et 12 V, pour engendrer le champ électrique.

3. Procédé selon la revendication 1 ou 2, caractérisé en ce que le mélange fait du substrat et du matériel microbien est additionné de polyélectrolytes et/ou d'échangeurs d'ions avant application du champ électrique.

4. Procédé selon l'une des revendications 1, 2 ou 3, caractérisé en ce que le champ électrique est appliqué par intermittence.

5. Procédé selon l'une des revendications 1 à 4, caractérisé en ce que le champ électrique est appliqué au moins pendant une première période partielle de la transformation au cours de laquelle le substrat est absorbé par le matériel microbien.

6. Procédé selon la revendication 5, caractérise en ce qu'une tension aux bornes supérieure à la tension de décomposition de la solution substrat-biomasse, est appliquée pour favoriser la flottation de la biomasse chargée du substrat.

7. Procédé selon l'une revendications 1 à 6, caractérisé en ce que l'on ajoute des substances nutritives au mélange, avant application du champ électrique, le pH étant le cas échéant amené à une valeur souhaitée.

8. Procédé selon l'une des revendications 1 à 7, caractérisé en ce que l'on applique un champ électrique aux lignes de champ à peu près horizontales.

9. Procédé selon l'une des revendications 1 à 8, caractérisé en ce que le chargement de la biomasse par le substrat et la transformation biologique de la biomasse chargée avec le substrat s'effectuent dans des récipients séparés, le matériel chargé avec le substrat étant séparé et acheminé vers une enceinte de réaction, en particulier un bassin de boues activées, et la biomasse est acheminée vers l'enceinte de mélange, après transformation dans l'enceinte de réaction, le cas échéant après aérage.

10. Procédé selon l'une des revendications 1 à 9, caractérisé en ce que dans l'enceinte de mélange et/ou dans le dispositif de flottation, les électrodes sont disposées à une distance l'une de l'autre qui varie entre 0,5 et 2 mm/V en fonction de la tension choisie entre 2 et 30 V, et qui est de préférence égale à 1 mm/V.

11. Procédé selon l'une des revendications 1 à 10, caractérisé en ce que l'écartement des électrodes dans le dispositif de flottation est choisi supérieur à l'écartement des électrodes dans l'enceinte de mélange et/ou la tension aux bornes dans l'enceinte de mélange est choisie supérieure à la tension aux bornes dans l'enceinte de réaction.

12. Procédé selon l'une des revendications 1 à 11, caractérisé en ce que le champ électrique est maintenu pendant une période comprise entre 2 et 10 min, de préférence énviron 5 min, dans le bassin de mélange ou bassin de flottation séparés de l'enceinte de réaction, en particulier du bassin de boues activées ou d'aérage.